Europäisches Patentamt

European Patent Office

Office européen des brevets

(11) Veröffentlichungsnummer: **0 346 321 B1**

(12) **EUROPÄISCHE PATENTSCHRIFT**

(45) Veröffentlichungstag der Patentschrift: **21.09.94**

(21) Anmeldenummer: **89890160.8**

(22) Anmeldetag: **08.06.89**

(51) Int. Cl.⁵: **A61B 6/03**

(54) **Verfahren zur Beseitigung von Störungen eines über einen Detektor aufgenommenen Röntgenbildes.**

(30) Priorität: **09.06.88 AT 1504/88**

(43) Veröffentlichungstag der Anmeldung:
**13.12.89 Patentblatt 89/50**

(45) Bekanntmachung des Hinweises auf die Patenterteilung:
**21.09.94 Patentblatt 94/38**

(84) Benannte Vertragsstaaten:
**AT BE CH DE ES FR GB IT LI LU NL SE**

(56) Entgegenhaltungen:
**EP-A- 0 153 786**

**IEEE TRANSACTIONS ON NUCLEAR SCIENCE, Band NS-23, Nr. 5, Oktober 1976, Seiten 1453-1458, New York US; J.P. STONESTROM et al.: "Scatter considerations in fan beam computerized tomographic systems"**

**PHYSICS MEDICINE AND BIOLOGY, Band 22, Nr. 2, März 1977, Seiten 229-244, The Institute of Physics, London GB, J.J.Battista et al: "Compton scatter imaging of transverse sections: correction for multiple scatter and attenuations"**

(73) Patentinhaber: **Siemens Aktiengesellschaft Österreich**
**Siemensstrasse 88 - 92**
**A-1210 Wien (AT)**

(72) Erfinder: **Zinterhof, Peter, Dr.**
**Beethoven Strasse 48**
**A-5020 Salzburg (AT)**
Erfinder: **Efinger, Helmut, Dr.**
**Siegfried-Marcus-Strasse 20**
**A-5020 Salzburg (AT)**
Erfinder: **Hagenauer, Helge**
**Scheibenweg 3**
**A-5020 Salzburg (AT)**

(74) Vertreter: **Atzwanger, Richard, Dipl.-Ing. Patentanwalt**
**Mariahilfer Strasse 1c**
**A-1060 Wien (AT)**

JOURNAL OF APPLIED PHOTOGRAPHIC EN-
GINEERING, Band 9, Nr. 6, Dezember
1983,Seiten 184-195, Society of Photographic
Scientists and Engineers, Easton,Pennsylvania, US; M.J. YAFFE et al.:
"Scattered radiation in diagnostic radiology:magnitudes, effects and methods of reduction"

Radiological Imaging Vol 1-2 P. 199, 629 - 646
Academic Press 1981

Radiation Dosimetry Academic Press 1968 P.
230 - 236

**Beschreibung**

Die Erfindung betrifft ein Verfahren zur Beseitigung von Störungen in einem über Detektoren aufgenommenen Röntgenbild, die durch vom Röntgenstrahl heim Durchdringen eines Körpers erzeugte Streustrahlung verursacht werden.

In der DE 26 13 809 C3 wird beschrieben, wie in der Röntgendiagnostik, insbesondere der Computer-Tomographie, Röntgenbilder auf elektronischem Wege erzeugt werden. Ein Detektor mißt die Intensität des einfallenden Röntgenstrahls nach der Durchstrahlung des Körpers. Über eine Meßwertabfrageeinheit und einen Rechner werden die Bildpunkte umgesetzt und an einem Fernsehsichtgerät dargestellt.

Die Auflösung und damit die Qualität des Bildes wird wesentlich durch die Streustrahlung beeinflußt, die der Röntgenstrahl beim Durchdringen des Körpers erzeugt. Zur Verbesserung der Bildqualität werden daher Streustrahlblenden verwendet, wie sie beispielsweise in der AT 339.440 beschrieben sind. Diese Streustrahlblenden beeinflussen aber selbst wieder durch die Abschattung des primären Röntgenstrahls die Bildqualität. Dieser Effekt kann durch Bewegung der Blende und eine Erhöhung der Röntgenenergie gemildert werden. Dabei wird aber der Körper des Patienten mit einer höheren Strahlungsdosis belastet und die Lebensdauer der Röntgenröhre verkürzt.

Der Erfindung liegt die Aufgabe zugrunde, die Bildqualität ohne Verwendung einer Streustrahlblende zu verbessern.

Dies wird mittels des Verfahrens gemäß Patentanspruch 1 gelöst.

Nach diesem Verfahren wird der durch die Streustrahlung erzeugte Meßfehler der Detektoren durch ein digitales Filter korrigiert. Die physikalische Analyse erfaßt das Streuungsverhaltens der Röntgenquanten genauer als bisher. Der Einfluß der ermittelten Streustrahlung wird nach der Ermittlung ihrer räumlichen Verteilung beim Bildaufbau berücksichtigt und weitgehend kompensiert. Je nach Anwendungsgebiet kann nicht nur das Bild einer Schicht, sondern durch Integration über alle drei Raumrichtungen auch die räumliche Auflösung eines Tomogramms verbessert werden. Im dreidimensionalen Fall wird nicht zwischen einzelnen Schichtbildern des durchstrahlten Körpers interpoliert, sondern auch der Einfluß der Streustrahlen von einer auf die nächste Schicht berechnet. Somit entfällt auch der Einfluß des Streustrahleffektes in der dritten Dimension.

Zur weiteren Auswertung der Meßwerte für beispielsweise quantitative Analyse des durchstrahlten Körpergewebes ist es vorteilhaft, daß aus der Punktstreuungsfunktion durch Integration über die Querschnittsfläche des Röntgenstrahls die Gesamtzahl der gestreuten Röntgenquanten in der Detektorfläche bestimmt wird. Weiters ist es vorteilhaft, daß die Streustrahlungsenergie pro Einheitselement in der Detektorfläche durch Multiplikation der Punktstreuungsfunktion mit der Energie gestreuter Röntgenquanten bestimmt wird. Das Streuverhalten von Körperteilen mit unterschiedlichen Massenschwächungskoeffizienten kann dadurch näher untersucht werden, daß die mittlere Energie der gestreuten Röntgenquanten in der Detektorfläche durch Division der über die Querschnittsfläche des Röntgenstrahls integrierten Streustrahlungsenergie durch die Gesamtzahl der gestreuten Röntgenquanten bestimmt wird. Eine weitere Verbesserung der Bildqualität wird dadurch erreicht, daß die Genauigkeit der Berechnung der Streuquanten in der Steuerformel durch höhere Korrekturterme der Reihenentwicklung des Streuoperators erhöht wird.

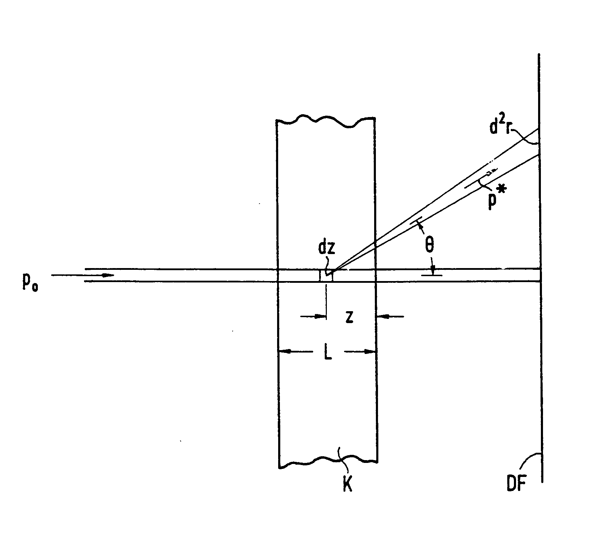

Die Erfindung wird anhand eines Ausführungsbeispieles und einer Zeichnung näher erläutert. Die Figur zeigt den Aufriß der den Berechnungen zugrundeliegenden Anordnung des Ausführungsbeispieles.

In der Röntgendiagnostik wird ein menschlicher Körper von einem Röntgenstrahl durchdrungen, der nach unterschiedlicher Schwächung durch einzelne Körperteile deren Abbild erzeugt. Mit einem Detektorfeld aus mehreren einzelnen Detektoren wird die Röntgenstrahlung in elektrische Signale umgewandelt. Anhand dieser Meßwerte werden beispielsweise in der Computer-Tomographie durch mathematische Faltungsoperationen Schichtbilder des Körpers erzeugt. Die Röntgenstrahlung wird jedoch nicht nur geschwächt, sondern auch gestreut. Dabei dominieren im Bereich niederer Energie bis etwa 40 keV photoelektrische Wechselwirkungen. Im für die Röntgendiagnostik wichtigen Bereich mittlerer Energie bis 150 keV überwiegt die Compton-Streuung. Da die Streustrahlung die Bildqualität beträchtlich vermindert, werden die Meßwerte der Detektoren entsprechend der Energie der gestreuten Röntgenstrahlung korrigiert. Anstelle einer Korrektur der Meßwerte können auch Detektoren verwendet werden, die im Streuenergiebereich unempfindlich sind, bzw. kann ein geeignetes Filter zwischen Körper und Detektor angebracht werden.

Die Korrektur basiert auf einer Analyse der Schwächung und Streuung des Röntgenstrahls. Die Streustrahlung verursacht den diffusen Bildanteil. Die Anzahl der gestreuten Röntgenquanten als Streuquanten $N_{scat}$ wird dabei aus einer Lösung der Transportgleichung vom Boltzmann-Typ gewonnen, die eine Verteilungsfunktion $f(\vec{p},\vec{r})$ im Phasenraum des Röntgenstrahls im durchstrahlten Körper beschreibt.

$$\hat{p} \cdot grad\ f(\vec{p}, \vec{r}) = -\mu f(\vec{p}, \vec{r}) + n_e \tilde{S} f(\vec{p}, \vec{r})$$

Die Schwächung der Röntgenstrahlung ist abhängig vom Massenschwächungskoeffizienten $\mu$. Die Compton-Streuung ist proportional der Zahl der im wesentlichen freien Elektronen $n_e$ und wird duch einen linearen Operator $\tilde{S}$ beschrieben. Die Rückstreuung der Röntgenquanten wird als linearer Effekt angenommen. Ein mit dem Streuoperator $\tilde{S}$ verbundener Kern s beschreibt die photoelektrische (elastische) - und Compton (inelastische) - Streuung

$$s(\vec{p}, \vec{p^*}) = \frac{1}{(p^*)^2} \delta\left(p - p^* + \frac{\Delta E(\theta)}{c}\right) \frac{d\sigma^c}{d\Omega}$$

$d\sigma^C/d\Omega$ ist der differentielle Wirkungsquerschnitt für Compton-Streuung, $\Delta E$ der Energieverlust durch inelastische Streuung, p,p* der Impuls des einfallenden und gestreuten Röntgenquants und c die Lichtgeschwindigkeit.

Zur exakten Lösung der Transportgleichung in geschlossener Form wird ein linearer Operator $\tilde{B}$ definiert, der den Streuoperator $\tilde{S}$ enthält.

$$f(\vec{p}, \vec{r}) = \tilde{B} f(\vec{p}, \vec{r_0}) \qquad\qquad \tilde{B} = e^{-\int_{r_0}^{r} (\mu I - n_e \tilde{S}) dl}$$

Weiters wird der Streuoperator $\tilde{S}$ in eine Potenzreihe entwickelt und das Medium als homogen angenommen. Die Potenzen des Streuoperators $\tilde{S}$ entsprechen mehrfachen Streuereignissen. Unter Einbeziehung der zweiten Näherung ergibt sich somit die Anzahl der Streuquanten je Volumenelement $d\tau$ aus

$$\frac{d^2 N_{scat}}{d\Omega d\tau} \cong \Phi_0 e^{-\mu l} n_e \frac{d\sigma^c}{d\Omega} \left\{ (1 - \mu l) \frac{1}{p_0^2} \left(p_0 - \frac{\Delta E}{c}\right)^2 + \mu^c l \left(1 - \frac{\mu l}{2!}\right) \frac{1}{p_0^2} \left(p_0 - 2\frac{\Delta E}{c}\right)^2 + \ldots \right\}$$

Da der Massenschwächungskoeffizient $\mu$ menschlicher Körperteile klein ist, werden Potenzierungen der Zahl der Elektronen $n_e$ vernachlässigt. Explizite Ausdrücke für den differenziellen Wirkungsquerschnitt und den Energieverlust durch inelastische Streuung können der Standardliteratur entnommen werden.

Die Fig. zeigt die den physikalischen Betrachtungen zugrundeliegende Anordnung. Eine Detektorfläche DF wird als eben angenommen, obwohl in der Praxis die Detektoren oft auf Kreisbögen angeordnet sind. Ein streuender Körper K wird als homogenes Medium mit einer Dicke L angenommen. Die einfallenden Röntgenquanten des primären Röntgenstrahls mit einem Impuls $p_o$ schließen mit dem gestreuten Röntgenstrahl mit einem Impuls p* einen Streuwinkel $\theta$ (infinitesimal $d\Omega$) ein. Daher ergibt sich für die Streuquanten die Streuformel

$$\frac{d N_{scat}}{d\Omega} = \Phi_0 e^{-\mu(L-z)} \frac{d\sigma^c}{d\Omega} n_e A dz \left\{ \frac{1}{p_0^2} \left(p_0 - \frac{\Delta E(\theta)}{c}\right)^2 + n_e \sigma^c (L-z) \frac{1}{p_0^2} \left(p_0 - 2\frac{\Delta E(\theta)}{c}\right)^2 + \ldots \right\},$$

wobei $\Phi_o$ der Photonenfluß des einfallenden Röntgenstrahls mit der Querschnittsfläche A ist. Das streuende Körpervolumen hat eine Länge dz und ist einen Abstand z vom Austrittspunkt des primären Röntgenstrahls entfernt. Die Näherung für Einfachstreuung ist durch den ersten Term des Klammerausdrucks gegeben. Eine Punktstreuungsfunktion $h_s(r)$ als Gesamtzahl der gestreuten Röntgenquanten je Detektorelement $d^2r$ ergibt sich aus

$$h_s(r)d^2r = \int_0^L \frac{dN_{scat}}{d\Omega} e^{-\mu z \sec \theta} \, d\Omega dz$$

Die Punktstreuungsfunktion ist ein Maß für die Bildgüte, da sie den diffusen Bildanteil repräsentiert. Vor der Bildrekonstruktion ist die Punktstreuungsfunktion so klein als möglich zu halten. Durch die Berücksichtigung der Gesamtzahl der gestreuten Röntgenquanten in der Detektorfläche DF durch

$$\int_A h_s(r)d^2r$$

wird die Bildqualität beträchtlich gesteigert. Die Streustrahlungsenergie $\epsilon(r)$ pro Einheitselement $d^2r$ in der Detektorfläche DF folgt aus

$$\epsilon(r)d^2r = \int_0^L h\nu^* \frac{dN_{scat}}{d\Omega} e^{-\mu z \sec \theta} d\Omega$$

Dabei ist $\nu^*$ die Frequenz der gestreuten Röntgenquanten und h das Planck'sche Wirkungsquantum.

Die mittlere Energie der gestreuten Röntgenquanten in der Detektorfläche DF zur Abschätzung des diffusen Bildanteils ergibt sich nach

$$h\nu_s = \frac{\int \epsilon(r)d^2r}{\int h_s(r)d^2r} \; .$$

Es tritt eine Frequenzverschiebung abhängig von der Dicke L des durchstrahlten Körpers K auf, da mit wachsender Dicke L des Körpers K der dem Detektor gegenüberliegende Winkel des Streukegels abnimmt. Gleiches gilt bei größerem Abstand des Körpers K vom Detektor. Bei wachsendem Streuwirkungsquerschnitt sinkt die mittlere Streufrequenz, die auch noch vom streuenden Material abhängig ist. Da bei leichterem Material mehr gestreute Photonen den Detektor erreichen, ist die Frequenzverschiebung ausgeprägter.

**Patentansprüche**

1. Verfahren zur Verbesserung der Qualität eines mittels Detektoren aufgenommenen Röntgenbildes durch Verminderung der in diesem infolge von Streuquanten enthaltenen Streustrahlung, wobei das Röntgenbild durch einen Körper (K) durchsetzende Röntgenstrahlen erzeugt wird und wobei die Anzahl pro Einheitswinkel derjenigen Streuquanten, welche von den einzelnen Volumselementen des durchstrahlten Körpers ausgehen, als Lösung der Transportgleichung vom Boltzmann-Typ dadurch rechnerisch ermittelt wird, daß die der Anzahl der Streuquanten pro Flächenelement in der Detektorebene entsprechende Punktstreuungsfunktion nach Multiplikation der Anzahl pro Einheitswinkel Streuquanten mit einem winkelabhängigen Massenschwächungsfaktor über den Weg des Röntgenstrahls durch den Körper (K) hindurch integriert wird, dadurch gekennzeichnet, daß die Streustrahlung ausgefiltert wird, wobei für diese Ausfilterung die Anzahl pro Einheitswinkel derjenigen Streuquanten, welche von den einzelnen Volumselementen des durchstrahlten Körpers ausgehen, als auch deren Frequenz durch Reihenentwicklung eines Streuoperators als Lösung der genannten Transportgleichung bestimmt werden.

**2.** Verfahren nach Patentanspruch 1, dadurch gekennzeichnet, daß aus der Punktstreuungsfunktion durch Integration über die gesamte Detektorfläche (DF) die Gesamtzahl der Streuquanten in der Detektorfläche (DF) bestimmt wird.

**3.** Verfahren nach einem der Patentansprüche 1 und 2, dadurch gekennzeichnet, daß die Energie der Streuquanten pro Flächenelement in der Detektorfläche (DF) durch Multiplikation der Punktstreuungsfunktion mit der Energie der Streuquanten bestimmt wird.

**4.** Verfahren nach einem der Patentansprüche 1 bis 3, dadurch gekennzeichnet, daß die mittlere Energie der Streuquanten in der Detektorfläche (DF) durch Division der über die Querschnittsfläche des Röntgenstrahls integrierten Streustrahlungsenergie durch die Gesamtzahl der gestreuten Röntgenquanten bestimmt wird.

**5.** Verfahren nach einem der Patentansprüche 1 bis 4, dadurch gekennzeichnet, daß die Genauigkeit der Berechnung der Streuquanten in der Streuformel durch höhere Korrekturterme der Reihenentwicklung des Streuoperators erhöht wird.

**Claims**

**1.** A method of improving the quality of an X-ray image taken by means of detectors by reducing the stray radiation contained therein as a result of stray quanta, the X-ray image being generated by X-rays passing through a body (K) and the number per unit angle of such stray quanta, which issue from the individual volume elements of the body radiated through, being evaluated mathematically as a solution to the transport equation of the Boltzmann type, in that the point stray function corresponding to the number of stray quanta per surface element in the detector plane is integrated after multiplication of the number per unit angle of stray quanta with an angle-dependent mass attenuation factor over the path of the X-ray through the body (K), characterized in that the stray radiation is filtered out, the number per unit angle of such stray quanta, which issue from the individual volume elements of the body radiated through, as well as the frequency thereof is determined for this filtering-out by series expansion of a stray operator as a solution to the above-mentioned transport equation.

**2.** A process according to claim 1, characterized in that the total number of stray quanta in the detector area (DF) is determined from the point stray function by integration over the entire detector area (DF).

**3.** A process according to either one of claims 1 and 2, characterized in that the energy of the stray quanta per surface element in the detector area (DF) is determined by multiplication of the point stray function with the energy of the stray quanta.

**4.** A process according to any one of claims 1 to 3, characterized in that the average energy of the stray quanta in the detector area (DF) is determined by division of the stray radiation energy integrated via the cross sectional area of the X-ray by the total number of stray X-ray quanta.

**5.** A process according to any one of claims 1 to 4, characterized in that the accuracy of calculation of the stray quanta in the stray formula is increased by relatively high correction terms of the series expansion of the stray operator.

**Revendications**

**1.** Procédé pour l'amélioration de la qualité d'une image radiologique prise au moyen de détecteurs, par la réduction du rayonnement dispersé contenu dans ladite image suite à la présence de quantas de dispersion, l'image radiologique étant générée par des rayons X traversant un corps (K), et le nombre des quantas de dispersion par unité d'angle qui partent des divers éléments volumique du corps irradié est calculé, en résolvant l'équation de transfert du type Boltzmann en intégrant la fonction de dispersion ponctuelle correspondant au nombre de quantas de dispersion par élément de surface dans le plan détecteur, après multiplication du nombre de quantas de dispersion par unité d'angle avec un facteur d'atténuation massique fonction de l'angle, sur le trajet du rayon X à travers le corps (K), caractérisé en ce que le rayonnement dispersé est filtré, ce filtrage impliquant la détermination, sous la forme de la résolution de l'équation de transfert mentionnée plus haut, du nombre par unité d'angle des quantas de dispersion qui partent des divers élément volumiques du corps irradié ainsi que leur fréquence par la génération en série d'un opérateur de dispersion.

2. Procédé selon la revendication 1, caractérisé en ce qu'à partir de la fonction de dispersion ponctuelle, est déterminé, par intégration sur la totalité de l'aire des détecteurs (DF), le nombre total des quantas de dispersion présents dans ladite aire des détecteurs (DF).

3. Procédé selon l'une des revendications 1 et 2, caractérisé en ce que l'énergie des quantas de dispersion par élément de surface dans l'aire des détecteurs (DF) est déterminée en multipliant la fonction de dispersion ponctuelle par l'énergie des quantas de dispersion.

4. Procédé selon l'une des revendications 1 à 3, caractérisé en ce que l'énergie moyenne des quantas de dispersion dans l'aire des détecteurs (DF) est déterminée en divisant l'énergie du rayonnement dispersé intégrée par la section du rayon X par le nombre total des quantas de rayons X dispersés.

5. Procédé selon l'une des revendications 1 à 4, caractérisé en ce que la précision du calcul des quantas de dispersion est augmentée dans la formule de dispersion en choisissant des termes de correction plus élevés pour le développement en série de l'opérateur de dispersion.